# EUROPEAN PATENT APPLICATION

(11) **EP 2 384 747 A2**
(43) Date of publication of application: **09.11.2011**
(21) Application number: 11164774.9
(22) Date of filing: 04.05.2011
(51) Int. Cl.: A61K 9/50, A61K 31/4439, A61P 1/04

(54) **Oral Tablet Compositions Of Dexlansoprazole**

(30) Priority: 05.05.2010 TR 201003557; 28.07.2010 TR 201006225; 23.08.2010 TR 201007007
(71) Applicant: Sanovel Ilac Sanayi ve Ticaret A.S., 34398 Istanbul (TR)
(72) Inventor: Cifter, Ümit, 34398, Istanbul (TR); Öner, Levent, Ankara 34398, Istanbul (TR); Terkinli, Alper, 34398, Istanbul (TR); Üzer, Ibrahim Murat, 34398, Istanbul (TR); Türkyilmaz, Ali, 34398, Istanbul (TR)
(74) Representative: Sevinç, Erkan

(57) **Abstract**

The present invention relates to oral tablet compositions of dexlansoprazole or pharmaceutically acceptable salts or hydrated forms thereof having a gradual release and furthermore directed to processes for the manufacture of the tablet composition and its use in the treatment of gastrointestinal disorders.

## Description

### Technical Aspect

The present invention relates to oral tablet compositions of dexlansoprazole or pharmaceutically acceptable salts or hydrated forms thereof having a gradual release and furthermore directed to processes for the manufacture of the tablet composition and its use in the treatment of gastrointestinal disorders.

### Background of the Invention

The active ingredient, dexlansoprazole is the R-enantiomer of lansoprazole which inhibits gastric acid secretion (a proton pump inhibitor). Its chemical name is (+)-2-[(*R*)-{[3-methyl-4-(2,2,2-trifluoroethoxy)pyridin-2-yl] methyl} sulfinyl]-1*H*-benzimidazole and its chemical structure is shown in the Formula 1.

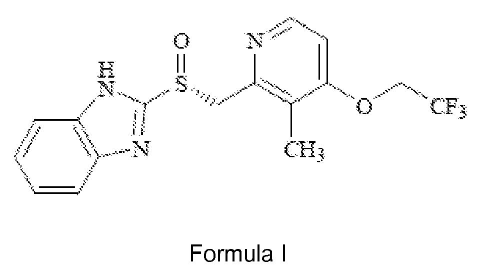

Delayed release capsule form of dexlansoprazole is in the market and it is administered orally in a therapeutic dose of 30 mg and 60 mg.

As other benzimidazole compounds, dexlansoprazole has also poor stability and is unstable to acidic medium, humidity, light and sensitive to heating. When orally administrated, it may not be able to sufficient activity since it is decomposed by gastric acid and the like. Thus, several problems occur in formulating such compound into oral pharmaceutical dosage forms because of the acidic environmental of the stomach. In particular, it will be rapidly decomposed and change colour under moist conditions or in acidic to neutral aqueous solution.

When these compounds are formulated into pharmaceutical preparations for oral administration, they require special techniques to avoid contact of drug with gastric acid of the stomach. One technique most commonly used is to coat these compounds, or its granules or pellets, with an enteric coating. However, the material used in enteric coatings itself is acidic, which can cause the decomposition of the compound. Such decomposition occurs even during the enteric coating process, which results in the coloration of the surface of the drug-containing core.

On the other hand, enteric films do not show high flexibility so that compression stress can yield rupturing of the film. It is therefore necessary to use a tableting technique that endorses the compression strain and maintains the acid resistance of the formulation after compression of the granules. Therefore caution is needed to be taken while compressing the powders and granules to form tablet dosage form. Such a formulation has to be compressed in a specific hardness.

In prior art, there are many patents including benzimidazoles such as lansoprazole and its R-enantiomer, dexlansoprazole in several different pharmaceutical compositions. Crystal form of R-lansoprazole is described in EP-B1-1129088.

Thus, there is still a need for developing pharmaceutical formulations of dexlansoprazole wherein a good stability is achieved in a technologically simple way and having improved manufacturing process which overcomes the above described problems and provides a bioavailable pharmaceutical composition according to the formulations currently used.

The pharmaceutical formulation of this invention advantageously provides a tablet dosage form which is bioequivalent to a capsule dosage form of the same or substantially similar strength. The tablet dosage form can further be advantageous in that the manufacturing process can require fewer steps, e.g., eliminate the need for pellet formation and/or coating of those pellets, and there is no need for the additional expense of providing capsule shells.

Further advantages and embodiments of the present invention will become apparent from the following description.

### Description of the invention

The main object of the present invention is to provide stable oral tablet compositions of dexlansoprazole or pharmaceutically acceptable salts or hydrated forms thereof having gradual release previously undisclosed in the prior art which overcomes the above described problems and have additive advantages over them.

Another object of the present invention is to express a pharmacological effect of the active ingredient stably and rapidly after administration and sustains pharmacological effect of gradual release for a prolonged period of time and to have a desired release profile, wherein the release of the active ingredient, dexlansoprazole, is controlled in two steps, and the active ingredient is released in the gastrointestinal tract over a long period of time. In prior art this sustained release is obtained mostly with using different enteric coatings comprising different polymers which dissolves in different pH. Because when the granules comprising the active ingredient first reach the proximal small intestine has a rapid release at pH 5,5 and the rest dissolves in distally in the small intestine at pH 6.5 to obtain the porolonged release.

Yet another object of the present invention is to provide an improved and simple process for preparing the oral tablet composition of dexlansoprazole or pharmaceutically acceptable salts or hydrated forms thereof having a gradual release.

In this invention, gradual release means that having two step release profile in a prolonged time. In first step 5.0 to 40.0 % of dexlansoprazole is release rapidly in its powder form from the oral tablet composition and have a stable effect up to 4 hours in proximal small intestine at pH 5.5, in second step the rest of the dexlansoprazole (60.0 to 95.0%) is released in a prolonged way in its granulation form from the oral tablet composition in small intestine at pH 6.0 to 6.5.

Surprisingly, we obtained this prolonged effect with gradual release of dexlansoprazole only using lactose and microcryistalline cellulose in granulation step of the oral tablet compositions of dexlansoprazole or pharmaceutically acceptable salts or hydrated forms thereof. More specifically, the weight ratio of lactose to microcrystalline cellulose in dexlansoprazole granule is between 1:20 and 1:5 by weight, preferably it is 1:9 by weight. Furthermore, the granule is comprising talc, colloidal silicon dioxide, magnesium stearate, hydrophobic and/or hydrophilic agents, an alkali compound, binders or their mixtures.

In other words, the desired gradual release achieved by not using pH dependent different coatings. This also prevents the dose dumping of the active ingredient which can be a serious problem caused by the wrong design of the modified release formulations.

Dose dumping is one of the most important disadvantages of modified release dosage forms. Because of several different reasons it is difficult to develop modified release formulations such as having a gradual release profile tablet formulations although there are many modified release formulations formulated with different coatings or using rate controlling agents. First of all, modified release formulations of these medicaments can be prone to "dose dumping" in which the release of the active ingredient is delayed but once the release begins the medicament may released very fast. The most important criter of dose dumping is the amount of the active substance released in early time point. Therefore, the active ingredient concentration in the plasma will increase suddenly and this may lead to toxicity.

According to the main object of the present invention, the oral tablet composition of dexlansoprazole or pharmaceutically acceptable salts or hydrated forms thereof having a gradual release characterised in that said tablet is comprising;
i. 5.0 to 40.0 % of dexlansoprazole in powder form,
ii. 60.0 to 95.0 % of dexlansoprazole granule comprising lactose, microcrystalline cellulose, a hydrophobic and/or hydrophilic agent, an alkali compound, binders or their mixtures,
iii. a single enteric coating that dissolves at between pH 5.5 and 6.4.

In one embodiment, the single enteric coating dissolves preferably at between pH 6.0 and 6.4.

According to another object of this present invention, to have a desired release profile and an improved stability and maximise the mechanical resistance of the tablets, this oral tablet formulation having a gradual release has been designed to compress in a specific hardness to form the tablets wherein the compression force of the powder and granule mixture of dexlansoprazole is in between 2 to 30 kN, preferably between 3 to 12 kN.

According to main object of the present invention the hydrophobic and/or hydrophilic agents in the dexlansoprazole granule wherein the amount is in between 60.0 to 95.0 % by weight, are selected from the group comprising hydrogenated vegetable oils such as hydrogenated castor oil; glyceryl behenate, wax, wax-like substance, fats, oils, fatty acid, fatty alcohol, shellac, pullulan, agar, gellan gum, guar gum, carageenan, acacia gum, gum arabic, dextran, pectin and their mixtures, preferably the hydrophobic agent is hydrogenated vegetable oils such as hydrogenated castor oil.

In one embodiment, the single enteric coating of the tablet that dissolves between at pH 5.5 and 6.4 is selected from the group comprising of cellulose acetate phthalate, cellulose acetate succinate, hydroxpropyl cellulose phthalate, hydroxpropyl ethylcellulose phthalate, hydroxyl propyl methyl cellulose phthalate, hydroxyl propyl methyl cellulose acetate succinate, hydroxyethyl cellulose phthalate, methylcellulose phthalate, polyvinyl acetate phthalate, polyvinylacetate hydrogen phthalate, amylase acetate phthalate, cellulose ester phthalates, cellulose ether phthalates, sodium cellulose acetate phthalate, starch acid phthalate, cellulose acetate butyrate, cellulose acetate maleate, cellulose acetate trimellitate, cellulose acetate propionate, styrene maleic acid dibutyl phthalate copolymer, styrene maleic acid polyvinyl acetate phthalate copolymer propionate, shellac and polymethacrylate copolymers such as methacrylic acid-ethyl acrylate copolymer, methacrylic acid- methyl methacrylate copolymer or mixtures thereof.

In one embodiment, the amount of the single enteric coating layer is between 1% and 50% (w/w) of the total weight of the tablet; preferably it is 5% to 30% (w/w).

According to one embodiment, the amount of dexlansoprazole or pharmaceutically acceptable salts or hydrated forms thereof is from %5 to %50 by weight of the total tablet.

Yet, another embodiment of the invention is to have a film coating under the enteric coating of the tablet so as to prevent any problems which may occur during the shelf-life. The film coating layer is selected from the group comprising of polyvinyl alcohol, polyvinylpyrrolidone (PVP), hydroxypropyl cellulose, lowsubstituted hydroxypropyl cellulose, hydroxypropylmethyl cellulose, sodium carboxymethyl cellulose, methyl and ethyl cellulose, hydroxyethyl methylcellulose, polyethylene glycol (PEG), PVP/vinyl acetate copolymer, PVA/PEG copolymer, alginates, sugar, starch, sugar alcohols (such as D-mannitol, erythritol, etc) or mixtures thereof.

The oral tablet composition of this invention, comprise one or more pharmaceutically acceptable excipients selected from the group comprising binders, diluents and/or fillers, lubricants, glidants, disintegrants, basic stabilizers, coloring agents or flavoring agents.

Suitable binders may comprise but not limited to methyl cellulose, ethyl cellulose, hydroxypropyl methyl cellulose, hydroxypropyl cellulose, sodium carboxymethyl cellulose, polyvinyl pyrrolidone, polyvinyl alcohol, polymethacrylates, starch, gelatin, alginic acid, sucrose and the like and mixtures thereof.

Suitable diluents and/or fillers may comprise but not limited to microcrystalline cellulose, cellulose, lactose, starch, calcium phosphates, calcium sulphates, mannitol, glucose, sucrose, sorbitol and the like and mixtures thereof.

Suitable lubricants may comprise but not limited to stearic acid, magnesium, calcium or sodium stearate, sodium stearyl fumarate, talc, waxes, liquid paraffin, and the like and mixtures thereof.

Suitable glidants may comprise but not limited to talc, aluminium silicate, colloidal silicon dioxide, starch and the like and mixtures thereof.

Suitable disintegrants may comprise but not limited to alginic acid and salts, sodium carboxymethyl cellulose, hydroxypropyl methyl cellulose, starch, sodium starch glycolate, crosslinked polyvinyl pyrrolidone and the like and mixtures thereof.

The oral tablet compositions of this invention are administrated once-a-day or twice-a-day.

In one embodiment, the oral tablet compositions of dexlansoprazole or pharmaceutically acceptable salts or hydrated forms thereof having a gradual release of this invention is obtained by the invitro dissolution profiles tested.

In this present invention, surprisingly the problem is also solved by more efficient process to prepare a gradual release oral tablet composition of dexlansoprazole or pharmaceutically acceptable salts or hydrated forms thereof comprising the following steps;
a. 5.0 to 40.0 % of dexlansoprazole is separated in its powder form,
b. 60.0 to 95.0 % of dexlansoprazole is blended in a powder blender with lactose and microcrystalline cellulose, an alkali compound, a hydrophobic and/or hydrophilic agent and binder,
c. this powder mix (step b) is then granulated using water, ethanol or an ethanol-water mixture,
d. wet granules are sieved and dried at a temperature not exceeding 45°C,
e. the granules are then sieved to obtain a maximum size of 500µm,
f. the granules are optionally blended with other excipients, such as magnesium stearate, colloidal silicon dioxide or talc,
g. dexlansoprazole (step a) in powder form is added to this granules and blended together until a homogenous powder mixture is obtained,
h. resulting powder is compressed into tablets,
i. the tablets are optionally precoated with a protective layer,
j. a single enteric coating that dissolves between at pH 5.5 and 6.4, is then applied to the precoated tablets.

The oral tablet composition of dexlansoprazole or pharmaceutically acceptable salts or hydrated forms thereof having a gradual release is used for the treatment of gastrointestinal disorders.

As apparent from the example below, by the method of the present invention the hardness of the tablet is improved. In addition, dissolution and stability is also improved.

This invention is further defined by reference to the following example. Although the example is not intended to limit the scope of the present invention, it should be considered in the light of the description detailed above. It will be apparent to those skilled in the art that many modifications, both to materials and methods, may be practiced without departing from the scope of the invention.

### Example 1

| **Ingredients** | **Amount (%)** |
|---|---|
| dexlansoprazole | 5.0-50.0 |
| lactose monohidrat | 10.0-95.0 |
| microcrystalline cellulose | 1.0-85.0 |
| magnesium oxide | 0.0-5.0 |
| talc | 0.0-10.0 |
| colloidal silicon dioxide | 0.0-10.0 |
| magnesium stearat | 0.0-15.0 |
| hydrogenated castor oil | 0.0-20.0 |
| Total | 100.0 |

Dexlansoprazole is blended in a powder blender till an adequate homogenous powder form is obtained and 5.0 to 40.0 % of it remains as in powder form. The rest of it (60.0 to 95.0 %) is blened together with lactose and microcrystalline cellulose in a dry powder blender for about 20 minutes, then an alkali compound, hydrophobic and/or hydrophilic agent and binder is added to this mixture and blended again. This this powder mix is then granulated using water, ethanol or an ethanol-water mixture and wet granules are sieved and dried at a temperature not exceeding 45°C. These granules are then sieved to obtained a maximum granule size of 500 µm. These granules are then blended with the rest of dexlansoprazole (5.0 to 40.0 %) powder and optionally with other excipients such as magnesium stearate, colloidal silicon dioxide or talc. The powder is then compressed into slugs using a suitable compression equipment and compression strength. The final blend is then compressed into tablets with a rotary tablet press using a compression strength of about 5 to 10 kN. These tablets are then coated in pan coater with the aforementined enteric coating that dissolves between at pH 5.5 and 6.4 and optionally coated with a protective layer coating up to an approximate weight gain of about 10%.

## Claims

1. An oral tablet of dexlansoprazole or pharmaceutically acceptable salts or hydrated forms thereof having a gradual release **characterised in that** said tablet is comprising;
i. 5.0 to 40.0 % of dexlansoprazole in powder form,
ii. 60.0 to 95.0 % of dexlansoprazole granule comprising lactose, microcrystalline cellulose, a hydrophobic and/or hydrophilic agent, an alkali compound, binders or their mixtures,
iii. a single enteric coating that dissolves at between pH 5.5 and 6.4.

2. The oral tablet according to claim 1, wherein the compression force to form the tablet is between 2 to 30 kN, preferably it is 3 to 12 kN.

3. The oral tablet according to claim 1, wherein the weight ratio of lactose to microcrystalline cellulose in dexlansoprazole granule is between 1:20 and 1:5 by weight, preferably it is 1:9 by weight.

4. The oral tablet according to claim 1, wherein the hydrophobic and/or hydrophilic agents are selected from the group comprising hydrogenated vegetable oils such as hydrogenated castor oil; glyceryl behenate, wax, wax-like substance, fats, oils, fatty acid, fatty alcohol, shellac, pullulan, agar, gellan gum, guar gum, carageenan, acacia gum, gum arabic, dextran, pectin and their mixtures.

5. The oral tablet according to claim 1, wherein the single enteric coating that dissolves at between pH 5.5 and 6.4 is selected from the group **comprising** of cellulose acetate phthalate, cellulose acetate succinate, hydroxpropyl cellulose phthalate, hydroxpropyl ethylcellulose phthalate, hydroxyl propyl methyl cellulose phthalate, hydroxyl propyl methyl cellulose acetate succinate, hydroxyethyl cellulose phthalate, methylcellulose phthalate, polyvinyl acetate phthalate, polyvinylacetate hydrogen phthalate, amylase acetate phthalate, cellulose ester phthalates, cellulose ether phthalates, sodium cellulose acetate phthalate, starch acid phthalate, cellulose acetate butyrate, cellulose acetate maleate, cellulose acetate trimellitate, cellulose acetate propionate, styrene maleic acid dibutyl phthalate copolymer, styrene maleic acid polyvinyl acetate phthalate copolymer propionate, shellac and polymethacrylate copolymers such as methacrylic acid-ethyl acrylate copolymer, methacrylic acid-methyl methacrylate copolymer or mixtures thereof.

6. The oral tablet according according to claim 1 and 5, wherein the amount of enteric coating layer is between 1% and 50% (w/w) of the total weight of the tablet, preferably it is 5% to 30% (w/w).

7. The oral tablet according to claim 1, further comprising a film coating under the enteric coating.

8. The oral tablet according to claim 7, wherein the film coating is selected from the group comprising of polyvinyl alcohol, polyvinylpyrrolidone (PVP), hydroxypropyl cellulose, lowsubstituted hydroxypropyl cellulose, hydroxypropylmethyl cellulose, sodium carboxymethyl cellulose, methyl and ethyl cellulose, hydroxyethyl methylcellulose, polyethylene glycol (PEG), PVP/vinyl acetate copolymer, PVA/PEG copolymer, alginates, sugar, starch, sugar alcohols (such as D-mannitol, erythritol, etc) or mixtures thereof.

9. A process for preparing the oral tablet of dexlansoprazole or pharmaceutically acceptable salts or hydrated forms thereof having a gradual release according to any preceding claims which comprises the following steps;
a. 5.0 to 40.0 % of dexlansoprazole is separated in its powder form,
b. 60.0 to 95.0 % of dexlansoprazole is blended in a powder blender with lactose and microcrystalline cellulose, an alkali compound and the hydrophobic and/or hydrophilic agent,
c. this powder mix (step b) is then granulated using water, ethanol or an ethanol-water mixture,
d. wet granules are sieved and dried at a temperature not exceeding 45°C,
e. the granules are then sieved to obtain a maximum size of 500µm,
f. the granules are optionally blended with other excipients, such as magnesium stearate, colloidal silicon dioxide or talc,
g. dexlansoprazole (step a) in powder form is added to this granules and blended together until a homogenous powder mixture is obtained,
h. resulting powder is compressed into tablets,
i. the tablets are optionally precoated with a protective layer,
j. a single enteric coating that dissolves between at pH 5.5 and 6.4, is then applied to the precoated tablets.

10. The oral tablet of dexlansoprazole or pharmaceutically acceptable salts or hydrated forms thereof having a gradual release according to any preceding claims, for use in the treatment of gastrointestinal disorders.
